(19) Europäisches Patentamt
European Patent Office
Office européen des brevets

(11) **EP 0 839 050 B1**

(12) **EUROPEAN PATENT SPECIFICATION**

(45) Date of publication and mention
of the grant of the patent:
**11.08.2004 Bulletin 2004/33**

(51) Int Cl.[7]: **A61K 39/00, A61K 39/02**

(21) Application number: **93921698.2**

(86) International application number:
**PCT/US1993/008900**

(22) Date of filing: **21.09.1993**

(87) International publication number:
**WO 1994/007531 (14.04.1994 Gazette 1994/09)**

(54) **METHOD OF ENHANCING CELL MEDIATED IMMUNE RESPONSES**

VERFAHREN ZUR VERSTÄRKUNG ZELLULARER IMMUNANTWORTEN

PROCEDE D'AMELIORATION DES REPONSES IMMUNITAIRES A MEDIATION CELLULAIRE

(84) Designated Contracting States:
**AT BE CH DE DK ES FR GB GR IE IT LI LU NL PT SE**

(30) Priority: **28.09.1992 US 952248**

(43) Date of publication of application:
**06.05.1998 Bulletin 1998/19**

(73) Proprietor: **Wyeth Holdings Corporation Madison, NJ 07940 (US)**

(72) Inventors:
• **BHOGAL, Balbir, S.**
**Lincoln, NE 68516 (US)**
• **DAYALU, Krishnaswamy, I.**
**Lincoln, NE 68506 (US)**
• **GERBER, Jay, D.**
**Lincoln, NE 68502 (US)**

(74) Representative: **VOSSIUS & PARTNER**
**Siebertstrasse 4**
**81675 München (DE)**

(56) References cited:
WO-A-91/18627          WO-A-92/03157
US-A- 4 894 332         US-A- 4 981 684
US-A- 4 985 243

• VETERINARNOMEDITSINSKI NAUKI, vol. 13, no. 9, 1976, pages 24-27, XP000614656 STOYANOV, V.: "Experiments on the preparation of vaccines against porcine enzootic pneumonia"
• THE FASEB JOURNAL, vol. 6, no. 5, 1992, page A1903 XP000614289 BHOGAL, B.S. ET AL.: "Preferential stimulation of cell mediated immune CMI responces in bronchial lymph nodes BLN of piglets vaccinated with a Mycoplasma hyopneumoniae (M.h.) vaccine"
• ANNALES DE L'INSTITUT PASTEUR, IMMUNOLOGY, vol. 138, no. 5, 1987, pages 693-705, XP000614309 KOBISCH, M. ET AL.: "The Mycoplasma hyopneumoniae plasma membrane as a vaccine against porcine enzootic pneumonia"
• AM. J. VET. RES., Volume 45, No. 10, issued October 1984, R.F. ROSS et al., "Characteristics of Protective Activity of Mycoplasma Hyopneumoniae Vaccine", pages 1899-1905.
• ACTA VETERINARIA (BEOGRAD), Volume 25, No. 4, issued 1975, S. DURISIC et al., "Antibodies in Blood, Colostral and Milk Sera of Sows Inoculated with an Experimental Vaccine of Mycoplasma Suipneumoniae", pages 189-194.

Remarks:
The file contains technical information submitted after the application was filed and not included in this specification

**Description**

This is a continuation-in-part of U. S. Patent application SN07/634,237, filed December 26, 1990, which is a continuation-in-part of U. S. Patent application SN07/575,921, filed August 31, 1990, which is a continuation-in-part of U. S. Patent application SN07/530,669, filed May 29, 1990.

Field of the Invention

The invention relates generally to the field of vaccines, and more specifically, to methods of enhancing cell mediated immune responses in newborn piglets to *Mycoplasma hyopneumoniae* antigens.

Background of the Invention

There have been documented instances in the art of passively immunizing neonates against infection with a selected disease-causing agent by administering a vaccine to a pregnant animal or to a nursing mother. These passive immune responses are believed to be due to the transfer of maternal IgA antibodies through the placenta to the infant and/or by intestinal absorption of maternal immunoglobulins present in the colostrum or milk by the neonate.

Colostrum and milk, which provide protective immune factors to the neonate, also have an extraordinary and unique combination of carbohydrates, fats, amino acids, minerals, vitamins, growth promoting factors such as epidermal-growth factor, insulin, and somatomedins, as well as lactoferrin, interleukin-1 (IL-1) and vasoactive intestinal peptides and some neuropeptides.

After ingestion of colostrum, maternal immune factor, especially antibodies, antibody producing cells, and T cells are seeded via intestinal mucosal tissue and maintained in various lymphoid tissues of the neonate during the early post-natal period of maturation of its immune system until the infant's own lymphoid system is capable of antibody production and induction or priming of T cells. Human colostrum and milk contain activated and memory T lymphocytes up to 73% of the total lymphocyte population. Memory T lymphocytes make up to 92% of the total lymphocyte population. Furthermore, experimental data on human colostrum and milk suggest that virtually all (99.8%) of T cells of the helper phenotype (CD4+), and most (92%) of the T cells of cytotoxic/suppressor phenotype (CD8+) are memory T cells. These T lymphocytes, in collaboration with other immune factors, play an important role in the immunological development of the neonate with respect to its ability to respond to future encounters with environmental antigens or deliberate exposure via vaccinations.

In the art of immunology it is well established that exposure of neonatal animals to a majority of environmental antigens or vaccination usually results in tolerance induction depending on various factors, such as type of antigen, dose and route of administration and genetic background. In contrast, a similar exposure of the adults would invariably result in an immune response.

The cellular basis of this disparate responsiveness of neonates and adults is not clearly defined. This phenomenon may be attributed to two critical factors, namely, status of maternal immune reactivity and maternal B and T cell repertoire, and the post-natal antigen exposure of the neonate by gram negative microbes (LPS). For example, colonization of the gut occurs rapidly after birth, e.g in calves about 4 days, and in pigs about 1 week.

Stoyanov, Veterinarnomeditsinski Nauki, 13(9), 1976, 24-27, discloses the vaccination of mother sows and subsequently of piglets with two different vaccines of M. hyorhinis.

There exists a need in the vaccine art for a method of enhancing immunity, particularly non-antibody mediated immunity (or cell mediated immunity), in neonates lacking fully developed immune systems.

Summary of the Invention

The present invention provides a method for enhancing cell mediated immune responses of newborn animals, particularly swine, to an antigen or immunogenic agent of *M. hyopneumoniae.* This method involves vaccinating pregnant animals with a vaccine containing the selected immunogenic agent and then administering a vaccine containing the same immunogen to the resulting newborns.

Other aspects and advantages of the present invention are described further in the following detailed description of the preferred embodiments thereof.

Detailed Description of the Invention

The present invention provides a method of enhancing cell mediated immune responses of newborn animals, particularly swine, to a *Mycoplasma hyopneumoniae* immunogen or antigen. This method involves administering to a pregnant sow a vaccine composition containing a selected *Mycoplasma* immunogenic agent and subsequently administering a suitable dose of the vaccine composition containing the same immunogen to the newborn piglet(s) to achieve a cell mediated immune response to that immunogen in the piglet.

This method elicits an enhanced cell mediated immune (CMI) response in the nursing and vaccinated newborns to the specific immunogen upon the second presentation of the immunogen through at least one direct vaccination of the piglet. Additionally, where a neonate is immunodeficient, the method of the invention, particularly via more than one vaccination of the nursing piglets, permits the maternal immune repertoire to be reconstituted in the newborn.

As used herein, the terms, *Mycoplasma hyopneumonia*e immunogen, immunogenic agent or antigen may refer to a whole *Mycoplasma hyopneumoniae* pathogen, preferably inactivated. These terms also include a pathogen protein isolated in crude form and/or purified therefrom or a synthetic protein, as well as some fragment of the synthetic, purified or isolated pathogenic protein having antigenic properties. It is possible that the antigen may also include a non-protein biological material from said pathogen.

By "enhanced CMI response" is meant a CMI response in the piglet comprising the production of T cells protective against the infectious agent bearing the antigen, which response is greater than that observed in pregnant sows induced by administration of the vaccine, or that observed when the vaccine is directly administered to a piglet only or that observed in unvaccinated piglets born to vaccinated sows. By "newborn" is meant a recently-born animal of less than about 10 weeks of age. Preferably in the method of this invention, the newborn has nursed on maternal colostrum after birth. Preferably, the newborn has nursed on maternal colostrum within the first 48 hours after birth. By "effective amount" or "effective immunogenic amount" as used herein is meant the amount of antigen which is capable of inducing in the vaccinated animal a protective cell-mediated immune response, and optionally, a protective antibody response. Alternatively, the effective amount may be defined as that required to prevent or lessen the severity, for some period of time, of any one of the disorders which result from infection with *M. hyopneumoniae*.

Thus, the present invention provides a method of enhancing cell mediated responses of piglets against a selected infectious agent, *M. hyopneumoniae.* The method involves administering a vaccine containing an effective amount of an antigen or immunogen from the infectious agent to a sow prior to birth of its piglets. According to the method of this invention, this vaccine can be administered to the sow prior to breeding. However, the vaccine is preferably administered to a pregnant sow between about 6 weeks and 2 weeks prior to farrowing, i.e., giving birth.

After birth, the piglets nurse on the milk and colostrum from the vaccinated mother, preferably within the first 48 hours after birth. It is preferred that the piglets nurse from the mother for a longer period of time. The piglet(s) then receive a primary vaccination, i.e., an appropriate first dose of the same vaccine composition that was administered to the mother. This primary vaccination is administered to the piglets at between 3 days and 3 weeks after birth. Preferably the primary vaccination is administered to the piglet one week from birth.

optionally, where desired, the vaccine is then administered to a piglet a second time, i.e., a secondary vaccination, approximately two weeks after the primary vaccination. The secondary vaccination can be desirable when the piglet is determined to be immunodeficient or when it has nursed insufficiently on material colostrum.

The immunoregulatory effect provided by the method of this invention is achieved even if the piglets are weaned after 48 hours. In the studies described below, the specific proliferative responses of T lymphocytes, that is, the cell mediated immune response, from piglets born to, and nursed by, sows vaccinated with the *Mycoplasma hyopneumoniae* vaccine were ten to twenty fold higher after the primary vaccination of the piglets than the responses of T lymphocytes from piglets born to, and nursed by, non-vaccinated sows after primary vaccination of these piglets.

While not wishing to be bound by the theory of the mechanism by which this method works, the inventors currently believe that colostrum of the previously vaccinated sows contain specific B cells and T cells carrying internal image antibodies to the antigen. The lymphoid organs of piglets nursing within at least the first 48 hours after birth on this colostrum are seeded with these maternal B and T cells.

Such lymphocytes colonize and proliferate in the piglet's lymphoid tissues and determine subsequent immunoreactivity of the piglet to the vaccine antigen. Upon exposure to the vaccine after birth, e.g., the primary vaccination, the piglet's immune system mounts an enhanced cell-mediated immune response to the immunogen in the vaccine.

In the course of a given immune response antibodies called idiotypes (Id or Ab-1), which have several immunogenic determinants formed by the interactions or folding of the hypervariable chains of Ab-1, are produced to a given antigen, in this case, the *M. hyopneumoniae* antigen. A second generation of immune responses, i.e., the generation of antibodies to the immunogenic determinants of Ab-1, also occurs as a part of the normal immunoregulatory circuits during the normal immune response. The antibodies generated to the Id determinants of Ab-1 during the course of an immune response to a given antigen are called anti-idiotypes (anti-Id or Ab-2). Several sets of Ab-2 antibodies, such as Ab-2β, Ab-2τ, and Ab-2α, can be produced against different idiotypic determinants of Ab-1. Some of the Ab-2, in particular Ab-28 produced against the Id located within the antigen binding site of Ab-1 antibodies, can bear a structural conformity to the antigen recognized by Ab-1. These Ab-2 antibodies can represent a mirror image or surrogate antigen to the host's immune system by virtue of their conformation, even though chemically they are proteins and not lipopolysaccharide (LPS) or LPS-like antigens. This forms the basis of idiotypic mimicry or internal imaging of a given antigen and provides a logical extension of the immune networks theory of Jerne, Ann. Immunol., 125(c):373 (1974)].

This system has a potential application in modulating immune responses, and may be useful in modulating the immunoresponsiveness of newborns. The immunization of pregnant animals induces a B cell response, e.g., immunoglobulin Ab-1, and a T cell response, such as T helper cells characterized by specificity for a given antigen. Studies in the murine system suggest that Lyb5$^-$ B lymphocytes and Lyb5$^-$ -like B lymphocytes, even though they are prone to tolerance induction by LPS-like antigens, can be very effectively stimulated for a positive and productive immune response by mirror-image surrogate antigens such as those represented by anti-idiotypic (Anti-Id) antibodies. In addition, Anti-Id has also been shown to enhance development of Lyb5$^+$ B subset of lymphocytes. Therefore the most practical natural means by which: (1) tolerization of Lyb5$^-$ B lymphocytes and Lyb5$^-$-like B lymphocytes can be prevented and (2) development of Lyb5$^+$ B lymphocytes can be enhanced in the newborn is via maternal vaccination and priming of the Lyb5$^-$ subset of B cells with Anti-Id. In this way, the newborn's lymphoid organs are seeded with these cells via ingestion of colostrum before exposure of the newborn to environmental antigens.

As exemplified herein, immunization of pregnant sows (or sows prior to breeding) with a given antigen of a selected infectious agent, e.g., *M. hyopneumoniae,* induces Ab-1 antibodies which are specific for the epitopes on that pathogen. Ab-2 antibodies are also produced against the Id of Ab-1. Some of these Ab-2 or Anti-Id bear structural conformity to the *Mycoplasma* antigens or a particular epitope of that antigen. Both the Ab-1 and Ab-2 maternal antibodies are carried to the newborn by antibody producing lymphocytes (B lymphocytes) during ingestion of colostrum in the first few hours after birth. Sow colostrum contains as much as 30% lymphocytes w/v for the first 16 hours postpartum. B and T lymphocytes absorbed through the intestinal mucosa in the first few hours of the newborn's life destines the establishment of the future T and B cell repertoire in various lymphoid organs.

This process is controlled by several factors including the specificity of the lymphocyte repertoire of the colostrum (which reflect the immune reactivity and repertoire of the mother), immaturity of the newborn's own B cell repertoire, and exposure of the newborn to environmental or selected vaccine antigens.

vaccine compositions useful in the method of the invention may be prepared as pharmaceutical compositions containing an effective immunogenic amount of the selected immunogen, e.g., the inactivated *M. hyopneumoniae* virus described below, as an active ingredient in a nontoxic and sterile pharmaceutically acceptable carrier. Such an acceptable carrier may be readily selected by one of skill in the art, and is preferably an aqueous carrier. A variety of aqueous carriers may be employed, e.g., water, buffered water, 0.4% saline, 0.3% glycine, and the like. These solutions are sterile and generally free of particulate matter. These solutions may be sterilized by conventional, well known sterilization techniques.

Such a vaccine compositions useful in this method may comprise the inactivated vaccine component described above or other Mycoplasma immunogens from the same *Mycoplasma* species. Still other antigens suitable for administration to swine, which are not of *Mycoplasma* origin, may be included in the vaccine composition administered according to this method.

These antigens may be mixed with optional pharmaceutically acceptable auxiliary substances as required to approximate physiological conditions such as pH adjusting and buffering agents, preservatives, emulsifiers and the like. Alternatively or additionally, the inactivated *M. hyopneumoniae* may be admixed or adsorbed with a conventional adjuvant, e.g., Amphigen, mineral oil and lecithin, aluminum hydroxide, muramyl dipeptide, and saponins such as Quil A.

A preferred embodiment of the vaccine composition which may be administered according to the method of the invention contains an aqueous suspension or solution containing the inactivated P-5722-3 strain of *M. hyopneumoniae. M. hyopneumoniae* strain P-5722-3 was deposited with the American Type Culture Collection, 12301 Parklawn Dr., Rockville, MD, under Accession No. 55052.

The vaccine containing this strain is available commercially under the tradename RespiSure [SmithKline Beecham] and is the subject of pending U.S. Patent Application Ser. No. 07/634,237, filed Dec. 26, 1990, and corresponding published International application PCT/US91/03689, which are incorporated by reference herein. The vaccine strain is preferably buffered at physiological pH, in a form ready for injection.

For purposes of this invention, a desirable immunogenic amount of inactivated *M. hyopneumoniae* virus for vaccination of the sow or the newborn, when administered as the sole active ingredient in a vaccine composition is between 5 X 10$^8$ CCU and 5 X 10$^9$ CCU. In a vaccine composition containing additional antigenic components, the same immunogenic amount or a reduced amount of *M. hyopneumoniae* may be employed. For use in the method of this invention, it is preferred that the vaccine composition is in unit dosage forms, containing preferably about 2 mls, each dose containing the desired titer.

Other appropriate therapeutically effective doses can be determined readily by those of skill in the art based on the above immunogenic amounts, the condition being treated and the physiological characteristics of the animal. In the presence of additional active agents, these unit dosages can be readily adjusted by those of skill in the art.

According to the method of this invention, a desirable dosage regimen involves administration of one or two doses of desired vaccine composition to the sow, either pregnant or prior to breeding, where the antigenic content of each fraction is desirably as stated above, and at least one dose to the newborn to obtain the enhanced CMI response of this method. Preferably, where two or more doses are administered to the pregnant animal, the doses are administered at least two weeks apart. In the case of the *M. hyopneumoniae* vaccine of this invention, for primary immunization of pregnant swine, two doses are recommended approximately four weeks apart with the last dose administered two weeks before farrowing. A booster dose is recommended prior to each subsequent farrowing, when the interval between vaccinations is more than six months. After the birth of the neonates, primary immunization should be initiated between approximately 3 days and 3 weeks of age, preferably, one week of age.

The mode of administration of the vaccines of the invention may be any suitable route which delivers the vaccine to the host. However, the vaccine is preferably administered by intramuscular injection. Other modes of administration may also be employed, where desired, such as subcutaneously, intradermally or intravenously.

The following example of the invention is illustrative only and not intended to be limiting.

EXAMPLE 1: Vaccine Trial

Eight white landrace pregnant sows were vaccinated intramuscularly at a period estimated to be between approximately two and 6 weeks prior to farrowing with the RespiSure vaccine composition [SmithKline Beecham) in a 2 mL dose. A control group of 8 pregnant sows was used, which animals were never vaccinated against *M. hyopneumoniae.* Newborn piglets at between one and three weeks of age which were nursing, or had nursed from the mother for at least 48 hours from birth, from both vaccinated and control groups were then administered a primary vaccination intramuscularly with the same dose of the same vaccine composition.

Flow cytometry was performed according to manufacturer's instructions [FACSTAR[PLUS] (Becton Dickinson, San Jose, CA)]. The flow cytometer, equipped with a single argon ion laser, was used for phenotypic analysis of lymphocytes obtained from the piglets at different time intervals after vaccination. Phenotypic analyses of lymphoid cells by flow cytometry using monoclonals specific for cluster of differentiation (CD) markers such as CD4 and CD8 were used to assess the effects of vaccination on piglets at one week of age and to assess the priming of T cells of the CD4 and CD8 phenotypes. Murine monoclonal antibodies, anti-CD4 and anti-CD8, specific for swine T cell markers were obtained from the ATCC. Anti-CD2 was obtained from Dr Joan Lunney [USDA Beltsville].

The following Table I summarizes the effects of primary vaccination on the T cell profiles in peripheral blood lymphocytes of piglets.

TABLE I

| Group | Intervention: of Piglets | % Positive Cells | |
|---|---|---|---|
| | | CD4 | CD8 |
| 1 | Controls | 29 | 46 |
| | | 31 | 24 |
| | | 35 | 50 |
| | | 32 | 61 |
| | *Average:* | $\overline{32}$ | $\overline{45}$ |
| 2 | Vaccinated | 69 | 36 |
| | born & nursed | 72 | 16 |
| | by vaccinated | 70 | 31 |
| | sows | 83 | 40 |
| | *Average:* | $\overline{74}$ | $\overline{22}$ |
| 3 | Vaccinated | 26 | 42 |
| | Nursed by | 36 | 30 |
| | Non-vaccinated | 40 | 56 |
| | Sows | 29 | 67 |
| | *Average:* | $\overline{33}$ | $\overline{48}$ |

Mycoplasma antigens used in this study were prepared from the vaccine strain of mycoplasma as described in PCT/US91/03689, incorporated by reference herein.

The lymphoid tissues of the piglets were removed aseptically at necropsy. Single cell preparations were prepared in tissue culture media RPMI 1640 containing 10% fetal calf serum and Penstrep [Gibco]. Cells were seeded in 96 well plates and incubated with or without antigen and with or without mitogens, such as Concanavalin A (ConA), or T cell mitogens and lipopolysaccharide (LPS), or B cell mitogens. After 72 to 96 hours cells were labeled with $^3$H-

thymidine, and incubated further for 18 to 24 hours. Cells were harvested on filter papers and incorporation of thymidine measured on beta-counter (scintillation counter; cpm). The degree of proliferation of cells is expressed as counts per minute.

At two weeks after a single vaccination, T lymphocytes from the spleen (mean cpm $31 \times 10^{-3}$), peripheral and bronchial lymph nodes (mean cpm $8.6 \times 10^{-3}$), and peripheral blood (mean cpm $21 \times 10^{-3}$) of the piglets showed very high proliferative responses to mycoplasma antigens.

The following paragraphs illustrate the responses seen in bronchial lymph nodes (BLN), peripheral lymph nodes (PLN), spleen, and peripheral blood lymphocytes (PBLs) following primary and secondary immunization and following challenge.

A. Differences in *in vitro* proliferative responses of lymphoid cells from piglets born and nursed by vaccinated or non-vaccinated sows: 14 days after first or primary vaccination with Respisure at one week of age.

TABLE II

| Source of lymphoid cells | Antigen | Mean cpm($^3$H-thymidine incorporation) | |
| --- | --- | --- | --- |
| | | Piglets born to and nursed by vaccinated sows | Piglets born to and nursed by non-vaccinated sows |
| Lymph nodes (BLN) | M.hyopn. | 8,800 | 2,100 |
| Lymph nodes (PLN) | " | 8,600 | 2,400 |
| Spleen | " | 31,800 | 3,700 |
| PBLs | " | 21,300 | 2,400 |
| n= 12 -16 piglets | | | |

B. Differences in *in vitro* proliferative responses of lymphoid cells from piglets born and nursed by vaccinated or non-vaccinated sows: 7 days after second or secondary vaccination with Respisure at three weeks of age.

TABLE III

| Source of lymphoid cells | Antigen | Mean cpm($^3$H-thymidine incorporation) | |
| --- | --- | --- | --- |
| | | Piglets born to and nursed by vaccinated sows | Piglets born to and nursed by non-vaccinated sows |
| Lymph nodes (BLN) | M.hyopn. | 9,300 | 3,400 |
| Lymph nodes (PLN) | " | 10,100 | 2,100 |
| Spleen | " | 19,700 | 4,800 |
| PBLs | " | 28,300 | 32,400 |
| n= 12-16 piglets | | | |

The high degree of responsiveness of T lymphocytes to the vaccine antigens is associated with the presence of various mycoplasma-specific immunocompetent T cells, especially T helper cells, in the lymphoid organs of piglets born and nursed by vaccinated sows. Post-primary vaccination responses of T cells from piglets born to vaccinated sows were comparable to the responses typically expected from a second vaccination in a piglet. This finding suggests the presence of T helper cells with a history of prior exposure to *Mycoplasma* antigens or antigen-like molecules.

C. Differences in *in vitro* proliferative responses of lymphoid cells from piglets born and nursed by vaccinated or non-vaccinated sows: 10 days after second or secondary vaccination with Respisure at three weeks of age.

TABLE IV

| Source of lymphoid cells | Antigen | Mean cpm ($^3$H-thymidine incorporation) | |
| --- | --- | --- | --- |
| | | Piglets born to and nursed by vaccinated sows | Piglets born to and nursed by non-vaccinated sows |
| Lymph nodes (BLN) | M.hyopn. | 10,100 | 3,600 |
| Lymph nodes (PLN) | " | 14,700 | 3,100 |
| Spleen | " | 26,100 | 11,200 |
| PBLs | " | 26,300 | 37,400 |
| n=12 to 16 piglets | | | |

The responsiveness of lymphoid cells from vaccinated pigs of vaccinated sows were assessed at 7 and 10 days after secondary vaccination as described above. Only marginal increases were observed, suggesting that a second vaccination would be of particular benefit to piglets that do not ingest adequate amounts of immune colostrum/milk under field conditions. While these colostrum-limited pigs may be less responsive to first vaccination, they would be able to mount a protective CMI response to a second vaccination.

D. Differences in *in vitro* proliferative responses of lymphoid cells from piglets born and nursed by vaccinated or non-vaccinated sows: 4 days after challenge infection with 2 mls virulent *M. hyopneumoniae* administered intranasally 10 days after secondary vaccination with Respisure at three weeks of age.

TABLE V

| Source of lymphoid cells | Antigen | Mean cpm ($^3$H-thymidine incorporation) | |
| --- | --- | --- | --- |
| | | Piglets born to and nursed by vaccinated sows | Piglets born to and nursed by non-vaccinated sows |
| Lymph nodes (BLN) | M.hyopn. | 25,500 | 3,400 |
| Lymph nodes (PLN) | " | 5,700 | 3,000 |
| Spleen | " | 4,900 | 18,200 |
| PBLs | " | 4,700 | 2,400 |
| n=12 to 16 piglets | | | |

E. Differences in *in vitro* proliferative responses of lymphoid cells from piglets born and nursed by vaccinated or non-vaccinated sows: 7 days after challenge infection with virulent *M. hyopneumoniae* given 10 days after secondary vaccination with Respisure at three weeks of age.

TABLE VI

| Source of lymphoid cells | Antigen | Mean cpm($^3$H-thymidine incorporation) | |
| --- | --- | --- | --- |
| | | Piglets born to and nursed by vaccinated sows | Piglets born to and nursed by non-vaccinated sows |
| Lymph nodes (BLN) | M.hyopn. | 44,300 | 19,980 |
| Lymph nodes (PLN) | " | 5,200 | 2,600 |
| Spleen | " | 4,700 | 9,300 |
| PBLs | " | 4,100 | 2,200 |
| n= 12 to 16 piglets | | | |

F. Differences in *in vitro* proliferative responses of lymphoid cells from piglets born and nursed by vaccinated or non-vaccinated sows: 11 days after challenge infection with virulent *M. hyopneumoniae* given 10 days after secondary vaccination with Respisure at three weeks of age.

TABLE VII

| Source of lymphoid cells | Antigen | Mean cpm($^3$H-thymidine incorporation) | |
| --- | --- | --- | --- |
| | | Piglets born to and nursed by vaccinated sows | Piglets born to and nursed by non-vaccinated sows |
| Lymph nodes (BLN) | M.hyopn. | 5,600 | 20,200 |
| Lymph nodes (PLN) | " | 15,100 | 8,600 |
| Spleen | " | 14,700 | 10,100 |
| PBLs | " | 14.500 | 7,200 |
| n= 12 to 16 piglets | | | |

Whereas marked increases in the CMI responses were observed in lymphoid cells from bronchial lymph nodes (BLN) 4 and 7 days post challenge with virulent *M. hyopneumoniae,* CMI responses of splenic, PBLs and BLN lymphocytes were down regulated in that very low responses were observed on days 4 and 7 post infection. An increase in CMI responses in these compartments was observed on day 11 post challenge using conventional techniques. However by this time, BLN lymphocytes from placebo animals challenge-infected in a similar manner also showed high CMI responses.

Although the example provided herein demonstrates this method with a particular *M. hyopneumoniae* vaccine administered to pigs, this invention is not limited thereby. The vaccine composition may contain other *Mycoplasma* antigens, and other species of *Mycoplasma* antigens. Additionally, the vaccine composition may contain combinations of *Mycoplasma* antigens, including antigens from the same species or different species of *Mycoplasma.*

It-is anticipated that this method of the invention can be used to enhance newborn CMI responses to other vaccinal proteins derived from pathogenic microorganisms or viruses, such as, feline infectious peritonitis virus, feline immunodeficiency virus, bovine rotavirus, canine parvovirus, *Borrelia*, and bovine *P. haemolytica.* It is anticipated to be especially useful in any animal species, particularly mammals, including humans, feline, canine and bovine, and in avian species, particularly poultry, including chicken and turkeys.

**Claims**

1.    Use of a Mycoplasma hyopneumoniae immunogen for the preparation of a vaccine composition for administration to both a female swine prior to the birth of its newborn and to its newborn after birth.

2.    The use of claim 1 wherein said vaccine composition is for administration to said female swine during its pregnancy.

3.    The use of claim 2 wherein said vaccine composition is for administration to said female swine between about 2 and about 6 weeks prior to giving birth.

4.    The use according to claim 1 wherein a vaccine composition is for first administration to the newborn about 3 days to about 3 weeks after birth.

5.    The use according to claim 4 wherein a second vaccine composition is for administration to the newborn about two weeks following said first administration.

6.    The use of any one of claims 1 to 5 wherein said immunogen is a whole inactivated Mycoplasma hyopneumoniae pathogen, a Mycoplasma hyopneumoniae protein isolated in crude form and/or purified therefrom or a synthetic Mycoplasma hyopneumoniae protein or a fragment of said synthetic, purified or isolated pathogenic protein having antigenic properties from said pathogen.

7.    The use of any one of claims 1 to 6 wherein said newborn prior to its vaccination has been nursed on the colostrum of said female swine.

8. The use of any one of claims 1 to 7 wherein, in addition to said M.hyopneumoniae immunogen, an antigen from a different species of Mycoplasma is to be administered.

**Patentansprüche**

1. Verwendung eines Mycoplasma hyopneumoniae-Immunogens für die Herstellung einer Impfstoffzusammensetzung zur Verabreichung an ein weibliches Schwein vor der Geburt seines Neugeborenen und an sein Neugeborenes nach der Geburt.

2. Verwendung nach Anspruch 1, wobei die Impfstoffzusammensetzung für die Verabreichung an ein weibliches Schwein während seiner Trächtigkeit ist.

3. Verwendung nach Anspruch 2, wobei die Impfstoffzusammensetzung für die Verabreichung an ein weibliches Schwein zwischen etwa 2 und 6 Wochen vor der Geburt ist.

4. Verwendung nach Anspruch 1, wobei eine Impfstoffzusammensetzung für die erste Verabreichung an das Neugeborene etwa 3 Tage bis 3 Wochen nach der Geburt ist.

5. Verwendung nach Anspruch 4, wobei eine zweite Impfstoffzusammensetzung für die Verabreichung an das Neugeborene etwa 2 Wochen nach der ersten Verabreichung ist.

6. Verwendung nach einem der Ansprüche 1 bis 5 wobei das Immunogen ein vollständiges, inaktives Mycoplasma hyopneumoniae-Pathogen, ein Mycoplasma hyopneumoniae-Protein, das in Rohform gewonnen und/oder daraus aufgereinigt wurde, oder ein synthetisches Mycoplasma hyopneumoniae-Protein oder ein Fragment des synthetischen, aufgereinigten oder gewonnenen pathogenen Proteins ist, welches die antigenen Eigenschaften des Pathogens besitzt.

7. Verwendung nach einem der Ansprüche 1 bis 6, wobei das Neugeborene vor der Impfung mit Kolostrum des weiblichen Schweins gesäugt wurde.

8. Verwendung nach einem der Ansprüche 1 bis 7, wobei, zusätzlich zu dem M. hyopneumoniae-Immunogen, ein Antigen einer anderen Mycoplasmaart verabreicht werden soll.

**Revendications**

1. Utilisation d'un agent immunogène de Mycoplasma hyopneumoniae pour la préparation d'une composition de vaccin destinée à l'administration à la fois à une truie avant la naissance de son nouveaux-né et à son nouveaux-né après la naissance.

2. Utilisation suivant la revendication 1, dans laquelle ladite composition de vaccin est destinée à l'administration à ladite truie au cours de sa gestation.

3. Utilisation suivant la revendication 2, dans laquelle ladite composition de vaccin est destinée à l'administration à ladite truie environ 2 à environ 6 semaines avant la mise bas.

4. Utilisation suivant la revendication 1, dans laquelle une composition de vaccin est destinée à l'administration tout d'abord au nouveau-né environ 3 jours à environ 3 semaines après la naissance.

5. Utilisation suivant la revendication 4, dans laquelle une seconde composition de vaccin est destinée à l'administration au nouveau-né environ 2 semaines après ladite première administration.

6. Utilisation suivant l'une quelconque des revendications 1 à 5, dans laquelle ledit agent immunogène est un agent pathogène Mycoplasma hyopneumoniae inactivé entier, une protéine de Mycoplasma hyopneumoniae isolée sous forme brute et/ou purifiée de cet agent pathogène ou une protéine synthétique de Mycoplasma hyopneumoniae ou un fragment de ladite protéine pathogène synthétique, purifié ou isolé ayant des propriétés antigéniques dudit agent pathogène.

**7.** Utilisation suivant l'une quelconque des revendications 1 à 6, dans laquelle ledit nouveau-né, avant sa vaccination, a été nourri avec le colostrum de ladite truie.

**8.** Utilisation suivant l'une quelconque des revendications 1 à 7, dans laquelle, en plus dudit agent immunogène de M. hyopneumoniae, un antigène provenant d'une espèce différente de Mycoplasma doit être administré.